# EUROPEAN PATENT APPLICATION

(11) **EP 4 847 058 A1**
(43) Date of publication of application: **02.12.2026**
(21) Application number: 26163524.7
(22) Date of filing: 10.03.2026
(51) Int. Cl.: A61M 1/16, A61M 1/26, A61M 1/36, A61M 60/38, A61M 60/113, A61M 60/268, A61M 60/837, A61M 39/00, A61M 39/08

(54) **GAS EXCHANGE TUBING**

(30) Priority: 10.03.2025 US 202563769450 P
(71) Applicant: Abiomed, Inc., Danvers, MA 01923 (US)
(72) Inventor: LUDDY, Charles, Danvers, 01923 (US)
(74) Representative: Wittmer, Maximilian

(57) **Abstract**

A device for blood-gas exchange is provided, as well as a system, kit, and method that utilize the device. The device includes a first tubular body defining at least one lumen extending from a distal end to a proximal end, and one or more hollow fibers extending at least partially between the distal end to the proximal end within the at least one lumen. Blood is configured to flow through the at least one lumen over at least a portion of the plurality of hollow fibers. A central, third tubular body may be provided, such that the hollow fibers are between the third tubular body and the first tubular body. The third tubular body may be configured to expand or contract radially as the pressure in the third tubular body changes. The flow of blood through the first lumen may be altered based on the pressure of the fluid flowing through the third lumen.

## Description

### CROSS-REFEENCE TO RELATED APPLICATIONS

The present application claims priority to U.S. Provisional Patent Application No. 63/769,450 filed March 10, 2025, the contents of which are incorporated by reference herein in its entirety.

### TECHNICAL FIELD

The present disclosure is drawn to devices, systems, and method for blood-gas exchange, and in particular, extracorporeal blood-gas exchange tubing.

### BACKGROUND

Extracorporeal blood-gas exchange systems have existed for decades. Modern approaches use extracorporeal membrane oxygenation (ECMO) devices to oxygenate blood. For example, Veno-Arterial Extracorporeal Membrane Oxygenation (VA-ECMO) and Veno-Venous Arterial Extracorporeal Membrane Oxygenation (VVA-ECMO) may involve the use of mechanical circulatory devices for patients experiencing oxygenation issues (e.g., for lunch support). In some instances, ECMO may be used for patients experiencing oxygenation issues due to cardiogenic shock, or other forms of hemodynamic deterioration.

### BRIEF SUMMARY

Disclosed is a device, system, kit, and method that provides a simple gas exchange system requiring minimal priming volume.

In some embodiments, a device for blood-gas exchange is provided. The device may include a first tubular body having a first end, a second end, and a first lumen extending therethrough, where the tubular body configured to transport blood and a sweep gas, the first tubular body further configured to allow at least one gas to transfer between the sweep gas and the blood as the blood passes through the first tubular body. The device may include one or more connectors configured to allow blood to enter the first end of the first tubular body and allow a sweep gas to enter the second end of the first tubular body. The device may include a first connector configured to allow blood to enter the first end of the first tubular body, the first connector being connected at or near the first end of the tubular body. The device may include a second connector configured to allow a sweep gas to enter the second end of the figure tubular body, the second connector being connected at or near the second end of the tubular body. The device may include a second tubular body positioned within at least the first lumen of the first tubular body, the second tubular body having a second lumen extending therethrough. The second tubular body may include a plurality of hollow fiber windings. In various arrangements, the total surface area of the plurality of hollow fiber windings in the device is at least 1.5 m², such as between 1.5 m² and 2.5 m². The plurality of hollow fiber windings may be configured to transport a gas in a first direction from the first end to the second end. The device may be configured to transport blood in a second direction from the second end to the first end, the second direction being opposite to the first direction.

The device may include a third tubular body positioned within the first tubular body such that the second tubular body is between the first tubular body and the third tubular body, the third tubular body having a lumen extending therethrough, the third tubular body configured to expand and/or contract radially as a pressure within the third lumen varies.

The device may be free of any housing around the first tubular body.

In some embodiments, a device for blood-gas exchange may be provided that includes a first tubular body defining at least one lumen extending from a distal end to a proximal end, a plurality of hollow fibers positioned within the at least one lumen, where the plurality of hollow fibers extending at least partially between the distal end to the proximal end, and where the at least one lumen is configured to allow blood to flow through the at least one lumen over at least a portion of the plurality of hollow fibers. The hollow fibers may be arranged in various configurations, and in some embodiments, the plurality of hollow fibers may include a plurality of fiber windings. In some embodiments, each of the plurality of hollow fibers may include a tubular body. In some embodiments, each of the plurality of hollow fibers is, independently, formed in a spiral pattern around a central axis. In various arrangements, the total surface area of the plurality of hollow fiber windings in the device is at least 1.5 m², such as between 1.5 m² and 2.5 m².

A sweep gas may be configured to flow through each of the plurality of hollow fibers. The sweep gas may be configured to flow in a first direction from the first end to the second end. The device may be configured to transport blood in a second direction from the second end to the first end, the second direction being opposite to the first direction.

The device may include a third tubular body within the at least one lumen of the first tubular body, the third tubular body having a third lumen extending therethrough. The third tubular body may be configured to expand and/or contract radially as a pressure within the third lumen varies.

The device may include a coupling configured to allow a tube operably connected to a fluid source to be removably coupled to the third tubular body. The third tubular body may be configured to have a fluid (such as water or a compliance gas) flowing through the third tubular body, wherein the fluid has a temperature greater than the blood flowing through the at least one lumen.

The third tubular body may be configured to allow a medical device or sensor to be slidably inserted into or through the third tubular body.

The device may include a first connector coupled at or near a proximal end of the first tubular body, and a second connector coupled at or near a distal end of the first tubular body. The first connector may include a fourth tubular body configured such that at least a portion of the fourth tubular body is radially positioned between the first tubular body and at least some of the plurality of hollow fibers. The second connector may include a fifth tubular body configured such that at least a portion of the fifth tubular body is radially positioned between the first tubular body and at least some of the plurality of hollow fibers. In some embodiments, either the first connector or the second connector may be configured to allow blood to enter the first tubular body and the sweep gas to exit the first tubular body, and wherein the other of the first connector or the second connector may be configured to allow the blood to exit the first tubular body and the sweep gas to enter the first tubular body.

The device may include potting in contact with at least a portion of an internal surface of a first connector and/or a second connector, where at least a portion of each of the plurality of hollow fibers extends through the potting. The potting may be configured such that a surface of the potting may be adjacent to a port configured to allow blood to exit or enter the first connector and/or the second connector. The surface of the potting may be parallel to an axis passing through the center of the port.

The first connector may include a first cap coupled to a proximal end of the first connector. The second connector may include a second cap coupled to a distal end of the second connector. The first cap and the second cap may each include a gas port, wherein one of the first cap and the second cap may be configured to allow the sweep gas to flow through a gas port and into each of the plurality of hollow fibers, and the other of the first cap or the second cap may be configured to allow the sweep gas to flow from each of the plurality of hollow fibers and out through the gas port. The first cap, the second cap, or both may include a port configured to allow a compliance gas to enter or exit the device.

The device may include at least one coupling configured to removably connect the third tubular body to a tube operably connected to a compliance gas source.

In some embodiments, a system for blood-gas exchange is provided. The system may include an embodiment of a first device for blood-gas exchange as disclosed herein. A distal end of the first device, a proximal end of the first device, or both may be operably connected to a subject such that blood from the subject flows from the patient, through at least the first device, and back into the patient.

The system may include an embodiment of a second device for blood-gas exchange as disclosed herein, where at least some of the blood flowing from the patient enters the second device. In some embodiments, the first device and the second device may be connected serially. In some embodiments, the first device and the second device may be connected in parallel. The system may include a manifold operably connected to the first device and the second device. The system may include at least one valve positioned prior (e.g., upstream) to the first device, the second device, or both.

In some embodiments, the first device, the second device, or both may be removably attachable to the system. The system may include a first quick connector at the distal end of the first device, and a second quick connector at the proximal end of the first device.

The system may include a pump operably coupled to at least the first device and the subject, the pump configured to cause blood to flow from the subject and through the first device. The pump may be a centrifugal pump or an inline pump. The pump may be integrated into the first device. The pump may be remotely located from the first device.

The system may include an oxygenator operably connected to the first device. The system may include a heat exchanger operably connected to the first device. The system may include a controller operably coupled to the first device. The controller may be configured to, e.g., control the flow of a compliance gas into the first device.

In some embodiments, a kit may be provided. The kit may include a plurality of devices, each independently an embodiment of a device for blood-gas exchange as disclosed here. The kit may include an oxygenator. The kit may include a heat exchanger. The kit may include a controller. The kit may include a pump. The kit may include a manifold.

In some embodiments, a method for blood-gas exchange is provided. The method may include increasing the percentage of oxygen in a first portion of blood by flowing at least the first portion of blood and a sweep gas through a first device, which is an embodiment of a device for blood-gas exchange as disclosed herein. The method may include flowing the first portion of blood and a sweep gas through a second device, which is an embodiment of a device for blood-gas exchange as disclosed herein. The method may include flowing a second portion of blood through a second device at the same time the first portion of blood is flowing through the first device, the second device being an embodiment of a device for blood-gas exchange as disclosed herein.

The method may include flowing the blood through a heat exchanger before the blood flows through the first device, flowing the blood through a heat exchanger after the blood flows through the first device, or both.

The method may include flowing the blood through an oxygenator before the blood flows through the first device. The method may include flowing the blood through an oxygenator after the blood flows through the first device.

The method may include altering the flow of blood through the first device by adjusting the flow of a fluid, such as a compliance gas, into a third tubular body within the first device.

The method may include adjusting the temperature of the blood flowing through the first device by flowing, through the second tubular body, a fluid at a different temperature than the temperature of the blood. The method may include adjusting the temperature of the blood flowing through the first device by flowing, through the third tubular body, a fluid at a different temperature than the temperature of the blood.

### BRIEF DESCRIPTION OF DRAWINGS

Figure 1A is an axial cross-sectional view of an embodiment of a device.
Figure 1B is a radial cross-sectional view of an embodiment of a device.
Figure 1C is an isometric view of a portion of a device.
Figure 2A and 2B are schematics of some embodiments of a system.
Figure 3 is a flowchart of an embodiment of a method.

### DETAILED DESCRIPTION

Extracorporeal blood-gas exchange systems have existed for decades. Modern approaches use extracorporeal membrane oxygenation (ECMO) devices to oxygenate blood. However, the use of centrifugal pumps and ECMO may be limited and/or difficult to deploy, such as, due to complications and/or complexity of the systems.

In view of the above, and other difficulties that may be experienced with ECMO circuits, the inventor has recognized the benefit of integrating gas exchange fibers into the form factor of ECMO tubing. In this regard, and as described herein, the disclosed system may create a blood conduit with a high length to diameter aspect ratio, allowing for gas transfer along the length of tubing. The inventor has also recognized the disclosed embodiments may allow for minimal priming volume (which, in some embodiments, may improve incidents of hemodilution) and/or faster deployment with quick priming (e.g., with air being removable from the circuit more quickly). The disclosed embodiments also may provide a longer path length of gas exchange fibers, which may maximize the contact time between the blood and the fibers, which in some embodiments may optimize gas exchange performance. In some embodiments, the disclosed embodiments may minimize or even eliminate stagnation zones since flow of blood through the tubing may be linear.

As will be appreciated, existing ECMO circuits also may be complex, there may be multiple functions integrated into a single tubing. In some instances, devices may fail to supply a pulsatile flow. Moreover, these systems may require a dedicated technician during the pump run, which may last for several days. Using the disclosed system, in some embodiments, there may not be a need for additional ECMO circuit components, since tubing is already used in ECMO circuits and the disclosed tubing may replace all or at least part of traditional ECMO tubing. This may make the ECMO circuit less intimidating users or to users that use ECMO less frequently. The disclosed tubing also may be able to take on multiple functions, as disclosed herein, with or without an integrated pump, and with varying lengths for different patients.

A device for blood-gas exchange may be provided, that offers ease of use, control, and reduced priming volumes.

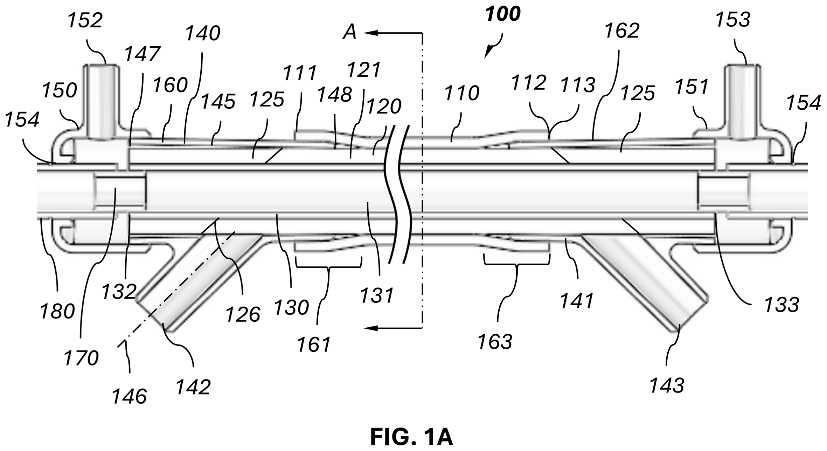

Referring to FIG. 1A, in some embodiments, a device **100** may include a first tubular body **110** having a first end **111** (which may be, *e.g.*, a proximal end or a distal end), a second end **112** opposite the first end (which may be, *e.g*., a distal end or a proximal end, as appropriate), and a first lumen **113** extending therethrough.

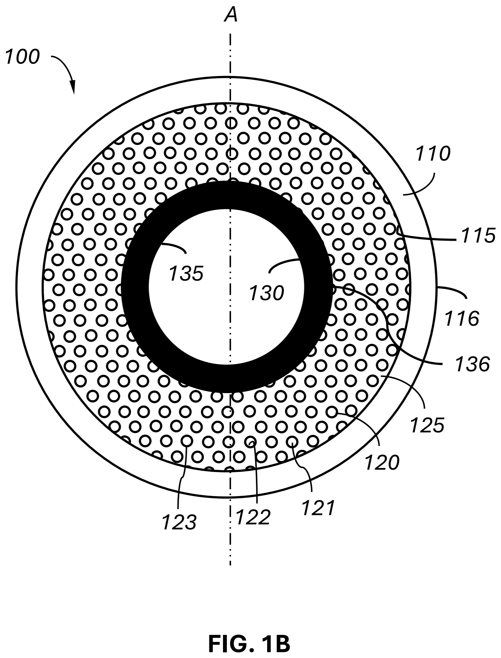

As seen in FIG. 1B, the first tubular body **110** may have an outer surface **116** and an inner surface **115,** the inner surface defining at least one lumen, including the first lumen. The first tubular body may be configured to transport blood and a sweep gas and may be configured to allow at least one gas to transfer between the sweep gas and the blood as the blood passes through the first tubular body.

In some embodiments, the device may be free of any housing around (e.g., external to) the first tubular body. In some embodiments, the device may be free of any housing coupled to an external surface of the first tubular body.

In some embodiments, the device may include a second tubular body **120** positioned within at least the first lumen **113** of the first tubular body **110.** The second tubular body the second tubular body having a second lumen **121** extending therethrough. In some embodiments, the second tubular body may include a plurality of hollow fibers, such as a plurality of hollow fiber windings, each having an inner surface **122** defining the second lumen **121** therethrough, and an outer surface **123.** The plurality of hollow fibers may be an array of hollow fibers.

The plurality of hollow fibers **120** may be positioned within the first lumen **113,** where the plurality of hollow fibers extends at least partially between the distal end **112** to the proximal end **111** of the first tubular body **110.**

The second tubular body, such as the hollow fibers, may be comprised of a gas-permeable material configured to allow the transport of oxygen, carbon monoxide, and carbon dioxide through the material.

It is this portion of the device, that includes the second tubular body, where the blood-gas exchange may occur. The first tubular body and the second tubular body may be configured to allow blood to flow through the first lumen over at least a portion of the second tubular body (which may be, e.g., over at least a portion of the plurality of hollow fibers). In some embodiments, the plurality of hollow fiber windings may be configured to transport a gas in a first direction from the first end **111** to the second end **112.** The gas would flow through the lumen in each hollow fiber. The flow of the gas and blood in the device may be configured as parallel flow or counterflow as desired. In some embodiments, the device may be configured to transport blood in the first direction. In some embodiments, the device may be configured to transport blood in a second direction from the second end to the first end, the second direction being opposite to the first direction.

In some embodiments, the total external surface area of the plurality of hollow fiber windings in the device may be at least 1.5 m². In some embodiments, the total surface area of the plurality of hollow fiber windings in the device may be between 1.5 m² and 2.5 m².

In some embodiments, the second tubular body or bodies may be configured to have a fluid (e.g., a sweep gas) flowing through the second tubular body, wherein the fluid has a temperature greater than the blood flowing through the at least one lumen. In this manner, heat may be transferred through the second tubular body to the blood within the first lumen, the blood being in contact with an outer surface of the second tubular body.

The device may include one or more connectors **140, 141.** The connectors may be configured to allow blood to enter into a first end **111** of the first tubular body **110,** and/or configured to allow a sweep gas to enter a second end **112** of the first tubular body **110.** In some embodiments, the first connector may be coupled at or near the first end (e.g., a proximal end) of the first tubular body, and the second connector may be coupled at or near the second end (e.g., a distal end) of the first tubular body.

In some embodiments, the connectors may include a first connector **140** that may be configured to allow blood to enter the first end **111** of the first tubular body **110,** the first connector being connected at or near the first end of the tubular body. In some embodiments, a portion of the first connector may be positioned within the first lumen **113.** In some embodiments, the first connector may be configured to allow a sweep gas to enter the first end of the first tubular body. In some embodiments, the first connector may be configured to allow a sweep gas to exit the first end of the first tubular body.

In some embodiments, the first connector includes a fourth tubular body **160.** The fourth tubular body may be configured such that at least a portion **161** of the fourth tubular body may be radially positioned between the first tubular body and at least some of the plurality of hollow fibers.

In some embodiments, the second connector includes a fifth body **162.** The fifth tubular body may be configured such that at least a portion **163** of the fifth tubular body may be radially positioned between the first tubular body and at least some of the plurality of hollow fibers.

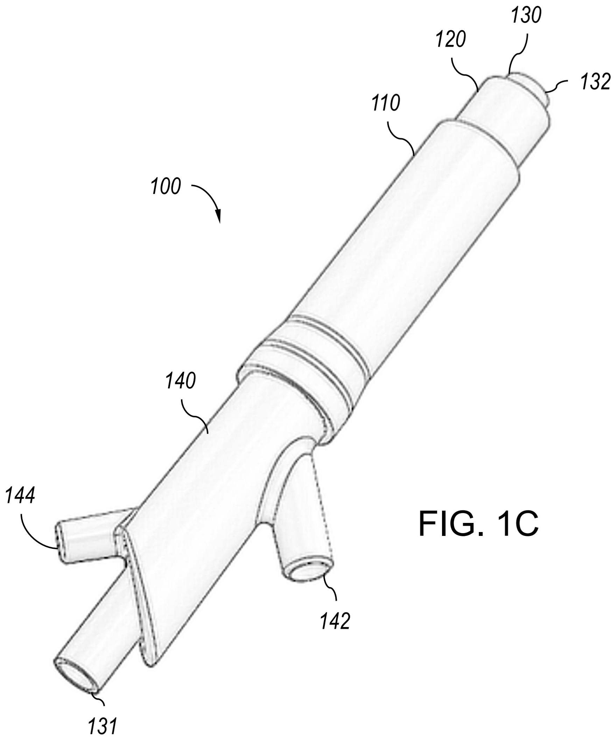

In some embodiments, at least one connector may include one or more side arms operably connected to the first lumen **113** and/or the second lumen **121.** The side arms may be configured to direct blood into or out of the first lumen, and/or direct a gas into or out of the second lumen. As seen in FIG. 1A, in some embodiments, one or more connectors may include a single side arm **142, 143.** In some embodiments, both connectors may include a single side arm. As seen in FIG. 1C, in some embodiments, one or more connectors may include two side arms **142, 144.** In some embodiments, both connectors may include two side arms.

In some embodiments, the one or more connectors may include a second connector 141 configured to allow a sweep gas to enter the second end **112** of the first tubular body, the second connector being connected at or near the second end of the tubular body. In some embodiments, a portion of the second connector may be positioned within the first lumen **113.** In some embodiments, the second connector may be configured to allow blood to enter the second end of the first tubular body. In some embodiments, the second connector may be configured to allow blood to exit the second end of the first tubular body.

In some embodiments, the device may include potting **125** in contact with at least a portion of an internal surface **145** of a connector (e.g., first connector **140** and/or second connector **141),** where at least a portion of each of the plurality of hollow fibers extends through the potting. This can be seen in FIG. 1B, where the plurality of hollow fibers **120** are positioned within potting 125.

In some embodiments, the potting **125** may extend from a first end **147** of a connector **140** towards a second end **148** of the connector. In some embodiments, the potting may be configured such that a surface **126** that blood may come into contact with may be adjacent to a port (such as a port extending through side arm **142)** configured to allow blow to exit or enter the connector (such as the first or second connector). In some embodiments, the surface **126** may be parallel to the plane defining the first end **147** of the connector. In some embodiments, the surface **126** may be parallel to an axis **146** passing through the center of the port.

In some embodiments, the device may include a third tubular body **130** positioned within the first tubular body **110.** The third tubular body may be positioned such that the second tubular body **120** may be between the first and third tubular bodies.

The third tubular body may include an inner surface **135** and an outer surface **136** and a sidewall therebetween. The inner surface may define a third lumen **131** extending from a first end **132** to a second end **133.**

The third tubular body may be configured to expand and/or contract radially as a pressure within the third lumen varies. For example, as a pressure within the third lumen increases, the third tubular body may expand radially, narrowing the distance between an outer surface **136** of the third tubular body and an inner surface **115** of the first tubular body.

In some embodiments, the device may include a coupling **170** configured to allow a tube **180** operably connected to a fluid source (not shown) to be removably coupled to the third tubular body **130.**

In some embodiments, a fluid flowing through this third lumen may be controlled in a manner such that the pressure in the third lumen periodically increases and decreases. By pulsing or otherwise controlling the pressure in the third lumen, the flow of blood within the first lumen may be controlled.

In some embodiments, the third tubular body may be configured to have a fluid flowing through the third tubular body, wherein the fluid has a temperature greater than the blood flowing through the at least one lumen. In this manner, heat may be transferred through the third tubular body to the blood within the first lumen, the blood being in contact with an outer surface of the third tubular body.

In some embodiments, the fluid may be a liquid. In some embodiments, the fluid may be water. In some embodiments, the fluid may comprise water (*e.g*., saline, etc.). In some embodiments, the fluid may be a gas. In some embodiments, the fluid may be a compliance gas.

In some embodiments, the third tubular body may be configured to allow a medical device or sensor to be slidably inserted into or through the third tubular body. In some embodiments, the inner surface **135** of the third tubular body may be coated to reduce friction. In some embodiments, the inner surface **135** defines an inner diameter that may be from 1 mm, 2 mm, or 3 mm up to 3 mm, 4 mm, 5 mm, 6 mm, 7 mm, 8 mm, 9 mm, or 10 mm, including all combinations and subranges thereof. In some embodiments, the inner diameter may be 1 mm - 9 mm.

The third tubular body may be comprised of an elastomeric material.

In some embodiments, the first connector may include a first cap **150** coupled to an end **147** (e.g., a proximal end) of the first connector. In some embodiments, the second connector may include a second cap **151** coupled to an end (e.g., a distal end) of the second connector. In some cases, both connectors have a cap on one end.

In some embodiments, the first cap **150** and the second cap **151** each further comprise a gas port **152, 153.** The first or second cap may be configured to allow the sweep gas to flow through a gas port (e.g., gas port **153** of second cap **151)** and into the second lumen (e.g., into each of the plurality of hollow fibers), while the other of the first cap or the second cap may be configured to allow the sweep gas to flow from each of the plurality of hollow fibers and out through the gas port (e.g., gas port **152** of first cap **150).**

In some embodiments, the first cap, the second cap, or both, may include at least one additional port **154, 155.** The at least one additional port may be configured to allow a fluid to enter or exit the device. In some embodiments, the fluid then enters the third lumen. The fluid may be any of the fluids described above with respect to the third lumen - water, a compliance gas, etc.

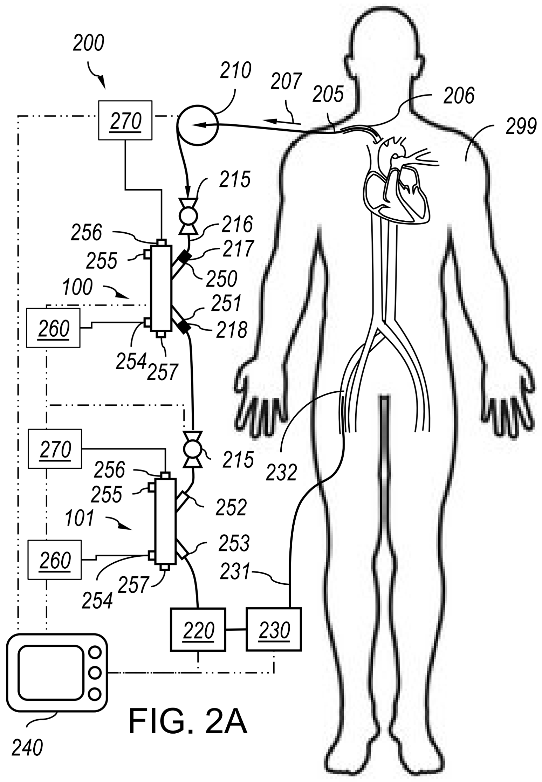

Referring to FIG. 2A, in some embodiments, a system **200** may be provided. In some embodiments, the system may include a first device **100** for blood-gas exchange as described herein.

In some embodiments, the first device may be part of a blood circuit, and operably connected to a patient (or subject). For example, in some embodiments, a first tube **205** may be inserted into a first blood vessel **206** (e.g., an artery) of a patient **299.** The patient's blood may then flow through the first tube **205** in a direction **207** from the patient, towards the first device **100.** The blood enters through a first end **250** (e.g., a proximal end) of the first device, through the first device, and out through a second end **251** (e.g., a distal end) of the first device. The blood may eventually flow into a second tube **231** that has been inserted into a second blood vessel **232** (e.g., a vein) of the patient **299.**

As seen in FIG. 2A, in some embodiments, a system may include a second device **101** for blood-gas exchange as described herein.

In some embodiments, the first device **100** and second device **101** are connected in series. That is, in some embodiments, a second end **251** (e.g., a distal end) of the first device **100** may be operably connected to a first end **252** (e.g., a proximal end) of the second device **101.**

In some embodiments, each device **100, 101** has its own sweep gas inlet **254** and sweep gas outlet **255.** In some embodiments, each sweep gas inlet **254** may be operably connected to a sweep gas source **260.** How a sweep gas passes through the system may vary. In some embodiments, the sweep gas exiting each sweep gas outlet **255** may be independently filtered and collected, or passed into the atmosphere. In some embodiments, a sweep gas outlet **255** from the second device **101** may be operably connected to a sweep gas inlet **254** of the first device **100.** In some embodiments, the sweep gas outlet **255** from the first device may be connected to a sweep gas inlet **254** of the second device **101.**

In some embodiment, each device **100, 101** has its own fluid inlet **256** and fluid outlet **257,** for allowing a fluid as described herein to pass through the device. In some embodiments, each fluid inlet **256** may be operably connected to a fluid source **270** via, *e.g.*, a coupling (see FIG. 1A, coupling **170).** How the fluid passes through the system may vary. In some embodiments, the system may be configured to run the fluid in series, where the fluid passes from the source, through one device, then through the next. In some embodiments, the system may be configured to run the fluid in parallel. While FIG. 2A shows two separate fluid sources **270,** it will be understood that in some embodiments, the system may be configured to utilize a single fluid source.

In some embodiments, at least one valve **215** may be positioned upstream / prior to the first device **100,** the second device **101,** or both. In some embodiments, the valve may be coupled directly to the first device and/or second device. In some embodiments, the valve **215** and the first device **100** and/or second device **101** are coupled together via tubing **216** and optionally a quick-disconnect fitting **217** at or near a first end **250** of the respective device.

In some embodiments, the system may include a first quick connector **217** at a first end **250** (e.g., proximal end) of the first device (and/or second device), and a second quick connector **218** at a second end **251** (e.g., a distal end) of the first device (and/or second device).

In some embodiments, the system may include a pump **210** operably connected to the first device. In some embodiments, the pump may be a centrifugal pump. In some embodiments, the pump may be an inline pump. In some embodiments, the pump may be integrated into the first device. For example, in some embodiments, an electrically-powered blood pump may be positioned in a side arm (see, *e.g*., FIG. 1A, ref. **142, 143).** Such blood pumps are well understood in the art. In some embodiments, the integrated blood pump may be a blood pump with a micromotor as described in, e.g., US 10,808,704. In some embodiments, the pump may be remotely located from the first device. In some embodiments, the pump may be remotely located from any device for blood-gas exchange.

In some embodiments, the pump **210** may be operably coupled to at least the first device and the subject. The pump may be configured to cause blood to flow from the subject and through at least the first device.

In some embodiments, the system may include an oxygenator **220** that may be operably connected to the first device. In some embodiments, the oxygenator may be located downstream (e.g., distally) from a first device. In some embodiments, the oxygenator may be located downstream from both a first and a second device. In some embodiments, the oxygenator may be located upstream (not shown) (e.g., proximally) from a first device. In some embodiments, the oxygenator may be located upstream from both a first and a second device.

In some embodiments, the system may include a heat exchanger **230** operably connected to the first device. In some embodiments, the heat exchanger may be located downstream (e.g., distally) from a first device. In some embodiments, the heat exchanger may be located downstream from both a first and a second device. In some embodiments, the heat exchanger may be located upstream (not shown) (e.g., proximally) from a first device. In some embodiments, the heat exchanger may be located upstream from both a first and a second device.

In some embodiments, the system may include a controller **240.** In some embodiments, the controller may be operably coupled to a first device.

In some embodiments, the controller may be configured to control the flow of a fluid from a fluid source **270,** such as a compliance gas, into the first device. In some embodiments, the controller may be configured to control the flow of a sweep gas from a sweep gas source **260,** into the first device. In some embodiments, the controller may be configured to control a pump **210.** In some embodiments, the controller v configured to control one or more valves **215.** In some embodiments, the controller may be configured to control an oxygenator **220.** In some embodiments, the controller may be configured to control a heat exchanger **230.**

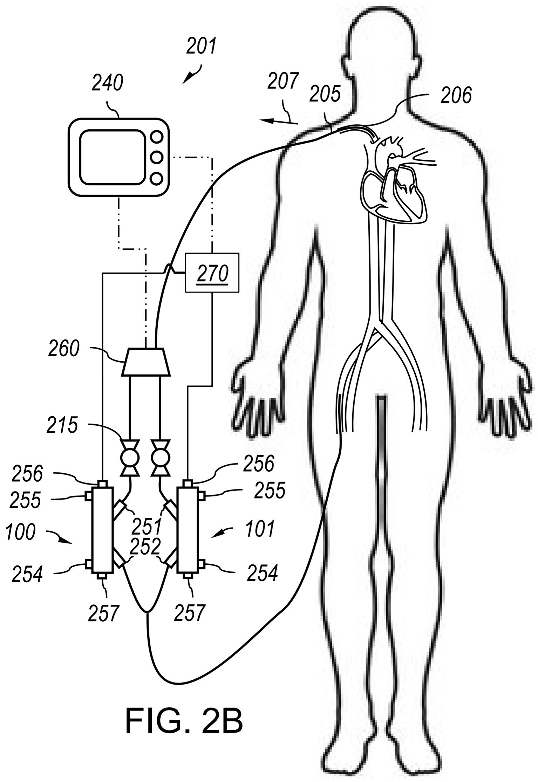

Referring to FIG. 2B, in some embodiments, the system **201** may be configured to have the first device **100** and the second device **101** be connected in parallel. In some embodiments, the system may include a manifold **260.** In some embodiments, the manifold may be operably connected to the first device and the second device. In some embodiments, a controller **240** may be configured to control the manifold **260.**

In some embodiments, a kit may be provided. In some embodiments, the kit may include a plurality of devices for blood-gas exchange as disclosed herein. In some embodiments, the kit may include an oxygenator. In some embodiments, the kit may include a heat exchanger. In some embodiments, the kit may include a pump configured to control the flow of blood through at least one of the plurality of devices. In some embodiments, the kit may include a controller. In some embodiments, the controller may be configured to control the flow of a fluid, such as a compliance gas, into the plurality of devices.

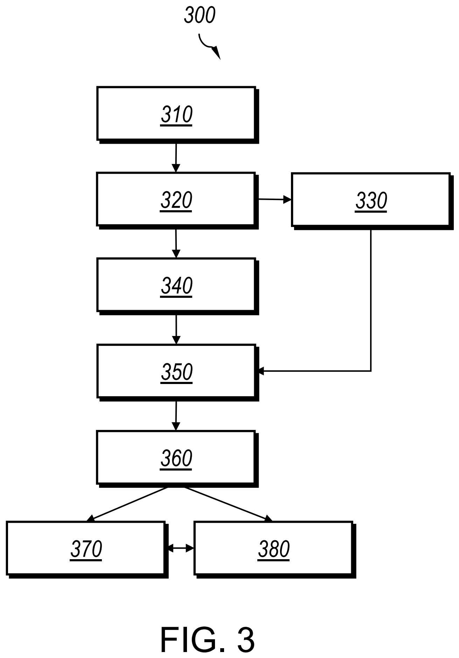

In some embodiments, a method may be provided. Referring to FIG. 3, in some embodiments, the method **300** may include increasing **320** the percentage of oxygen in a first portion of blood by flowing at least the first portion of blood and a sweep gas through a first device for blood-gas exchange as disclosed herein.

In some embodiments, the method may include flowing **330** a second portion of blood through a second device for blood-gas exchange as disclosed herein at the same time the first portion of blood may be flowing through the first device. This may occur if a system is configured to operate using parallel flow, such as if a manifold is used to direct some blood through a first device and other blood through a second device.

In some embodiments, the method may include flowing **340** the first portion of blood and a sweep gas through a second device for blood-gas exchange as disclosed herein. This may occur if a system is configured operate using serial flow.

In some embodiments, a separate heat exchanger may be utilized. Depending on the positioning of the heat exchanger relative to the blood-gas exchange devices, the method may vary accordingly. In some embodiments, the method may include warming the blood by flowing **310** the blood through a heat exchanger before the blood flows through the first device. In some embodiments, the method may include warming the blood by flowing **350** the blood through a heat exchanger after the blood flows through the first device.

In some embodiments, the method may include flowing **360** the blood through an oxygenator after the blood flows through the first device.

In some embodiments, the method may include altering **370** the flow of blood through the first device by adjusting the flow of a fluid (such as a compliance gas) into a third tubular body within the first device. For example, by pulsing the pressure within the tube, the flow of blood may be pulsed as well.

In some embodiments, the method may include adjusting **380** the temperature of the blood flowing through the first device. In some embodiments, this may be done by flowing, through the second tubular body, a fluid (such as a sweep gas) at a different temperature than the temperature of the blood. In some embodiments, this may be done by flowing, through the third tubular body, a fluid (such as the compliance gas) at a different temperature than the temperature of the blood.

Embodiments of the present disclosure are described in detail with reference to the figures wherein like reference numerals identify similar or identical elements. It is to be understood that the disclosed embodiments are merely examples of the disclosure, which may be embodied in various forms. Well known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

Those skilled in the art will recognize or be able to ascertain using no more than routine experimentation many equivalents to the specific embodiments of the invention described herein. Such equivalents are intended to be encompassed by the following claims.

### EXEMPLARY EMBODIMENTS

As already described, the technology described herein may be implemented in various ways. In that regard, the foregoing disclosure is intended to include, but not be limited to, the systems, methods, and combinations and subcombinations thereof that are set forth in the following exemplary implementations. Preferred embodiments are described in the following paragraphs:
A1 A device for blood-gas exchange, comprising:
   a first tubular body having a first end, a second end, and a first lumen extending therethrough, the tubular body configured to transport blood and a sweep gas, the first tubular body further configured to allow at least one gas to transfer between the sweep gas and the blood as the blood passes through the first tubular body.
A2 The device according to paragraph A1, further comprising one or more connectors configured to allow blood to enter the first end of the first tubular body; and
   configured to allow a sweep gas to enter the second end of the first tubular body.
A3 The device according to paragraph A2, wherein the one or more connectors includes a first connector configured to allow blood to enter the first end of the first tubular body, the first connector being connected at or near the first end of the tubular body.
A4 The device according to paragraph A3, wherein the one or more connectors includes a second connector configured to allow a sweep gas to enter the second end of the figure tubular body, the second connector being connected at or near the second end of the tubular body.
A5 The device according to any one of paragraphs A1-A4, further comprising a second tubular body positioned within at least the first lumen of the first tubular body, the second tubular body having a second lumen extending therethrough.
A6 The device according to paragraph A5, wherein the second tubular body includes a plurality of hollow fiber windings.
A7 The device according to paragraph A6, wherein the plurality of hollow fiber windings are configured to transport a gas in a first direction from the first end to the second end.
A8 The device according to paragraph A7, wherein the device is configured to transport blood in a second direction from the second end to the first end, the second direction being opposite to the first direction.
A9 The device according to any one of paragraphs A6-A8, wherein the total surface area of the plurality of hollow fiber windings in the device is at least 1.5 m².
A10 The device according to any one of paragraphs A6-A9, wherein the total surface area of the plurality of hollow fiber windings in the device is between 1.5 m² and 2.5 m².
A11 The device according to any one of paragraphs A1-A10, further comprising a third tubular body positioned within the first tubular body such that the second tubular body is between the first tubular body and the third tubular body, the third tubular body having a lumen extending therethrough, the third tubular body configured to expand and/or contract radially as a pressure within the third lumen varies.
A12 The device according to any one of paragraphs A1-A11, wherein the device is free of any housing around the first tubular body.
A13 A device for blood-gas exchange, comprising:
   a first tubular body defining at least one lumen extending from a distal end to a proximal end;
   a plurality of hollow fibers positioned within the at least one lumen, the plurality of hollow fibers extending at least partially between the distal end to the proximal end;
   wherein the at least one lumen is configured to allow blood to flow through the at least one lumen over at least a portion of the plurality of hollow fibers.
A14 The device according to paragraph A13, wherein the plurality of hollow fibers includes a plurality of fiber windings.
A15 The device according to paragraph A13, wherein each of the plurality of hollow fibers includes a tubular body.
A16 The device according to any one of paragraphs A13-A15, wherein a sweep gas is configured to flow through each of the plurality of hollow fibers.
A17 The device according to paragraph A16, wherein the sweep gas is configured to flow in a first direction from the first end to the second end.
A18 The device according to paragraph A17, wherein the device is configured to transport blood in a second direction from the second end to the first end, the second direction being opposite to the first direction.
A19 The device according to any one of paragraphs A13-A18, further comprising a third tubular body within the at least one lumen of the first tubular body, the third tubular body having a third lumen extending therethrough.
A20 The device according to paragraph A19, wherein the third tubular body is configured to expand and/or contract radially as a pressure within the third lumen varies.
A21 The device according to paragraph A19 or A20, further comprising a coupling configured to allow a tube operably connected to a fluid source to be removably coupled to the third tubular body.
A22 The device according to any one of paragraphs A19-A21, wherein the third tubular body is configured to have a fluid flowing through the third tubular body, wherein the fluid has a temperature greater than the blood flowing through the at least one lumen.
A23 The device according to paragraph A22, wherein the fluid is water.
A24 The device according to any one of paragraphs A13-A23, wherein the third tubular body is configured to allow a medical device or sensor to be slidably inserted into or through the third tubular body.
A25 The device according to any one of paragraphs A13-A24, further comprising a first connector coupled at or near a proximal end of the first tubular body, and a second connector coupled at or near a distal end of the first tubular body.
A26 The device according to paragraph A25, wherein the first connector includes a fourth tubular body configured such that at least a portion of the fourth tubular body is radially positioned between the first tubular body and at least some of the plurality of hollow fibers.
A27 The device according to paragraph A25 or A26, wherein the second connector includes a fifth tubular body configured such that at least a portion of the fifth tubular body is radially positioned between the first tubular body and at least some of the plurality of hollow fibers.
A28 The device according to any one of paragraphs A25-A27, wherein either the first connector or the second connector is configured to allow blood to enter the first tubular body and the sweep gas to exit the first tubular body, and wherein the other of the first connector or the second connector is configured to allow the blood to exit the first tubular body and the sweep gas to enter the first tubular body.
A29 The device according to any one of paragraphs A25-A28, further comprising potting in contact with at least a portion of an internal surface of a first connector and/or a second connector, where at least a portion of each of the plurality of hollow fibers extends through the potting.
A30 The device according to paragraph A29, wherein the potting is configured such that a surface of the potting is adjacent to a port configured to allow blood to exit or enter the first connector and/or the second connector.
A31 The device according to paragraph A30, wherein the surface of the potting is parallel to an axis passing through the center of the port.
A32 The device according to any one of paragraphs A25-A31, wherein the first connector further comprises a first cap coupled to a proximal end of the first connector, the second connector further comprises a second cap coupled to a distal end of the second connector, or both.
A33 The device according to paragraph A32, wherein the first cap and the second cap each further comprise a gas port, wherein one of the first cap and the second cap is configured to allow the sweep gas to flow through a gas port and into each of the plurality of hollow fibers, and the other of the first cap or the second cap is configured to allow the sweep gas to flow from each of the plurality of hollow fibers and out through the gas port.
A34 The device according to paragraph A33, wherein the first cap, the second cap, or both, further comprise an additional port configured to allow a compliance gas to enter or exit the device.
A35 The device according to paragraph A34, further comprising a coupling configured to removably connect the third tubular body to a tube operably connected to a compliance gas source.
A36 The device according to any one of paragraphs A5-A23, wherein each of the plurality of hollow fibers is, independently, formed in a spiral pattern around a central axis.
A37 The device according to any one of paragraphs A5-A24, wherein the total surface area of the plurality of hollow fibers in the device is at least 1.5 m².
A38 The device according to any one of paragraphs A5-A25, wherein the total surface area of the plurality of hollow fibers in the device is between 1.5 m² and 2.5 m².
A39 A system for blood-gas exchange, comprising:
   a first device according to any one of paragraphs A1-A38;
   wherein the distal end of the first device, the proximal end of the first device, or both are operably connected to a subject such that blood from the subject flows from the patient, through the first device, and back into the patient.
A40 The system according to paragraph A39, further comprising a second device according to any one of paragraphs A1-A38.
A41 The system according to paragraph A40, wherein the first device and the second device are connected in parallel.
A42 The system according to paragraph A41, further comprising a manifold operably connected to the first device and the second device.
A43 The system according to any one of paragraphs A40-A42, further comprising at least one valve positioned prior to the first device, the second device, or both.
A44 The system according to any one of paragraphs A40-A43, wherein the first device, the second device, or both are removably attachable to the system.
A45 The system according to paragraph A40, wherein the first device and the second device are connected serially.
A46 The system according to any one of paragraphs A39-A45, further comprising a first quick connector at the distal end of the first device, and a second quick connector at the proximal end of the first device.
A47 The system according to any one of paragraphs A39-A46, further comprising a pump operably coupled to at least the first device and the subject, the pump configured to cause blood to flow from the subject and through the first device.
A48 The system according to paragraph A47, wherein the pump is a centrifugal pump or an inline pump.
A49 The system according to paragraph A47 or A48, wherein the pump is integrated into the first device.
A50 The system according to any one of paragraphs A47-A49, wherein the pump is remotely located from the first device.
A51 The system according to any one of paragraphs A39-A50, further comprising an oxygenator operably connected to the first device.
A52 The system according to any one of paragraphs A39-A51, further comprising a heat exchanger operably connected to the first device.
A53 The system according to any one of paragraphs A39-A52, further comprising a controller operably coupled to the first device.
A54 The system according to paragraph A53, wherein the controller is configured to control the flow of a compliance gas into the first device.
A55 A kit comprising a plurality of devices according to any one of paragraphs A1-A38.
A56 The kit according to paragraph A55, further comprising an oxygenator.
A57 The kit according to paragraph A55 or A56, further comprising a heat exchanger.
A58 The kit according to any one of paragraphs A55-A57, further comprising a pump configured to control the flow of blood through at least one of the plurality of devices.
A59 The kit according to any one of paragraphs A55-A58, further comprising a controller configured to control the flow of a compliance gas into the plurality of devices.
A60 A method for blood-gas exchange, comprising:
   increasing the percentage of oxygen in a first portion of blood by flowing at least the first portion of blood and a sweep gas through a first device according to any one of paragraphs A1-A38.
A61 The method according to paragraph A60, further comprising flowing the first portion of blood and a sweep gas through a second device according to any one of paragraphs A1-A38.
A62 The method according to paragraph A60 or A61, further comprising flowing a second portion of blood through a second device according to any one of paragraphs A1-A38 at the same time the first portion of blood is flowing through the first device.
A63 The method according to any one of paragraphs A60-A62, further comprising flowing the blood through a heat exchanger before the blood flows through the first device.
A64 The method according to any one of paragraphs A60-A62, further comprising flowing the blood through a heat exchanger after the blood flows through the first device.
A65 The method according to any one of paragraphs A60-A64, further comprising flowing the blood through an oxygenator before the blood flows through the first device.
A66 The method according to any one of paragraphs A60-A64, further comprising flowing the blood through an oxygenator after the blood flows through the first device.
A67 The method according to any one of paragraphs A60-A66, further comprising altering the flow of blood through the first device by adjusting the flow of a compliance gas into a third tubular body within the first device.
A68 The method according to any one of paragraphs A60-A67, further comprising adjusting the temperature of the blood flowing through the first device by flowing, through the second tubular body, a fluid at a different temperature than the temperature of the blood.
A69 The method according to any one of paragraphs A60-A68, further comprising adjusting the temperature of the blood flowing through the first device by flowing, through the third tubular body, a fluid at a different temperature than the temperature of the blood.

## Claims

1. A device for blood-gas exchange, comprising:
a first tubular body having a first end, a second end, and a first lumen extending therethrough, the tubular body configured to transport blood and a sweep gas, the first tubular body further configured to allow at least one gas to transfer between the sweep gas and the blood as the blood passes through the first tubular body.

2. The device according to claim 1, further comprising one or more connectors configured to allow blood to enter the first end of the first tubular body; and
configured to allow a sweep gas to enter the second end of the first tubular body,
wherein the one or more connectors preferably includes a first connector configured to allow blood to enter the first end of the first tubular body, the first connector preferably being connected at or near the first end of the tubular body,
wherein the one or more connectors preferably includes a second connector configured to allow a sweep gas to enter the second end of the figure tubular body, the second connector preferably being connected at or near the second end of the tubular body.

3. The device according to claim 1 or 2, further comprising a second tubular body positioned within at least the first lumen of the first tubular body, the second tubular body having a second lumen extending therethrough.

4. The device according to claim 3, wherein the second tubular body includes a plurality of hollow fiber windings,
wherein the plurality of hollow fiber windings are preferably configured to transport a gas in a first direction from the first end to the second end, wherein the device is preferably configured to transport blood in a second direction from the second end to the first end, the second direction preferably being opposite to the first direction.

5. The device according to any one of claims 1-4, further comprising a third tubular body positioned within the first tubular body such that the second tubular body is between the first tubular body and the third tubular body, the third tubular body having a lumen extending therethrough, the third tubular body configured to expand and/or contract radially as a pressure within the third lumen varies,
and/ or wherein the device is free of any housing around the first tubular body.

6. A device for blood-gas exchange, comprising:
a first tubular body defining at least one lumen extending from a distal end to a proximal end;
a plurality of hollow fibers positioned within the at least one lumen, the plurality of hollow fibers extending at least partially between the distal end to the proximal end;
wherein the at least one lumen is configured to allow blood to flow through the at least one lumen over at least a portion of the plurality of hollow fibers,
wherein the plurality of hollow fibers preferably includes a plurality of fiber windings,
and/ or wherein each of the plurality of hollow fibers preferably includes a tubular body.

7. The device according to claim 6, wherein a sweep gas is configured to flow through each of the plurality of hollow fibers, wherein the sweep gas is preferably configured to flow in a first direction from the first end to the second end, and wherein the device is preferably configured to transport blood in a second direction from the second end to the first end, the second direction being preferably opposite to the first direction.

8. The device according to claim 6 or 7, further comprising a third tubular body within the at least one lumen of the first tubular body, the third tubular body having a third lumen extending therethrough, wherein the third tubular body is preferably configured to expand and/or contract radially as a pressure within the third lumen varies.

9. The device according to claim 8, further comprising a coupling configured to allow a tube operably connected to a fluid source to be removably coupled to the third tubular body,
and/ or wherein the third tubular body is configured to have a fluid flowing through the third tubular body, wherein the fluid has a temperature greater than the blood flowing through the at least one lumen,
and/ or wherein the third tubular body is configured to allow a medical device or sensor to be slidably inserted into or through the third tubular body.

10. The device according to any one of claims 6-9, further comprising a first connector coupled at or near a proximal end of the first tubular body, and a second connector coupled at or near a distal end of the first tubular body, wherein the first connector preferably includes a fourth tubular body configured such that at least a portion of the fourth tubular body is radially positioned between the first tubular body and at least some of the plurality of hollow fibers,
and/ or wherein the second connector includes a fifth tubular body configured such that at least a portion of the fifth tubular body is radially positioned between the first tubular body and at least some of the plurality of hollow fibers,
and/ or wherein either the first connector or the second connector is configured to allow blood to enter the first tubular body and the sweep gas to exit the first tubular body, and wherein the other of the first connector or the second connector is configured to allow the blood to exit the first tubular body and the sweep gas to enter the first tubular body,
and/ or further the device comprising potting in contact with at least a portion of an internal surface of a first connector and/or a second connector, where at least a portion of each of the plurality of hollow fibers extends through the potting, wherein the potting is preferably configured such that a surface of the potting is adjacent to a port configured to allow blood to exit or enter the first connector and/or the second connector, wherein the surface of the potting is preferably parallel to an axis passing through the center of the port.

11. The device according to claim 10, wherein the first connector further comprises a first cap coupled to a proximal end of the first connector, the second connector further comprises a second cap coupled to a distal end of the second connector, or both,
wherein the first cap and the second cap each preferably further comprise a gas port, wherein one of the first cap and the second cap is preferably configured to allow the sweep gas to flow through a gas port and into each of the plurality of hollow fibers, and the other of the first cap or the second cap is preferably configured to allow the sweep gas to flow from each of the plurality of hollow fibers and out through the gas port,
wherein the first cap, the second cap, or both, preferably further comprise an additional port configured to allow a compliance gas to enter or exit the device,
the device preferably further comprising a coupling configured to removably connect the third tubular body to a tube operably connected to a compliance gas source.

12. The device according to any one of claims 4-11, wherein each of the plurality of hollow fibers or hollow fiber windings is, independently, formed in a spiral pattern around a central axis,
and/ or wherein the total surface area of the plurality of hollow fibers or hollow fiber windings in the device is at least 1.5 m²,
and/ or wherein the total surface area of the plurality of hollow fibers or hollow fiber windings in the device is between 1.5 m² and 2.5 m².

13. A system for blood-gas exchange, comprising:
a first device according to any one of claims 1-12;
wherein the distal end of the first device, the proximal end of the first device, or both are operably connected to a subject such that blood from the subject flows from the patient, through the first device, and back into the patient.

14. The system according to claim 13, further comprising a second device according to any one of claims 1-12,
wherein the first device and the second device are preferably connected in parallel or serially,
and/ or the system preferably further comprising a manifold operably connected to the first device and the second device.

15. The system according to claim 13 or 14, further comprising at least one valve positioned prior to the first device, the second device, or both, and/ or
wherein the first device, the second device, or both are removably attachable to the system,
and/ or the system further comprising a first quick connector at the distal end of the first device, and a second quick connector at the proximal end of the first device,
and/ or the system , further comprising a pump operably coupled to at least the first device and the subject, the pump configured to cause blood to flow from the subject and through the first device, and/ or wherein the pump is preferably a centrifugal pump or an inline pump, and/ or wherein the pump is preferably integrated into the first device, and/ or wherein the pump is preferably remotely located from the first device,
and/ or the system further comprising an oxygenator operably connected to the first device,
and/ or the system further comprising a heat exchanger operably connected to the first device,
and/ or the system further comprising a controller operably coupled to the first device, wherein the controller is preferably configured to control the flow of a compliance gas into the first device.
